# EUROPÄISCHE PATENTANMELDUNG

(11) **EP 0 584 399 A1**
(43) Veröffentlichungstag der Anmeldung: **02.03.1994**
(21) Anmeldenummer: 92118805.8
(22) Anmeldetag: 03.11.1992
(51) Int. Cl.: A61H 39/00, A61N 1/20, A44C 7/00

(54) **Ohrschmuck zur Stimulation von Akupunktur-Punkten**

(30) Priorität: 26.08.1992 DE 4228342
(71) Anmelder: FIRMA FRANZ BREUNING, D-75172 Pforzheim (DE)
(72) Erfinder: Hieber, Fritz E.W., Dr. med., W-7570 Baden-Baden (DE)
(74) Vertreter: Trappenberg, Hans

(57) **Zusammenfassung**

Zur Anwendung der Elektro-Akupunktur wird eine umfangreiche Apparatur benötigt, die jedoch nur kurzzeitig die Akupunktur-Punkte reizen kann. Um eine stete Stimulierung dieser Akupunktur-Punkte zu erreichen, wird nach der Erfindung vorgeschlagen, den Strom einer in einem kreolenförmigen Ohrschmuck (1) befindlichen Stromquelle (5) über am Ohrschmuck angeordneten Kontaktflächen den nach der Lehre der Ohr-Akupunktur dort befindlichen Triggerpunkten zuzuleiten.

## Beschreibung

Die Phänomene der Akupunktur sind, obwohl die Akupunktur schon seit Jahrtausenden in Gebrauch ist, immer noch nicht vollständig geklärt. Bei der klassischen Akupunktur als Methode zur Erkennung und Heilung von Erkrankungen der Atmungs-, Kreislauf- und Verdauungsorgane, des Nervensystems und des Blutes, werden Nadeln aus unterschiedlichen Materialien an bestimmten, den jeweiligen Organen zugeordneten Hautstellen bis in die Unterhaut eingestochen. Dies soll der Beruhigung, Kräftigung und Wiederherstellung der Funktion des betroffenen Organs dienen. Bei der modernen Akupunktur werden ebenfalls die altüberlieferten Akupunktur-Punkte und -Linien gereizt beziehungsweise stimuliert, was sowohl durch Einstechen von Nadeln geschehen kann, wie aber auch durch Einwirkung von Druck, Wärme und elektrischem Strom. Die Erfindung bezieht sich auf die Reizung dieser Akupunktur-Punkte und -Linien durch elektrischen Strom, damit also auf die sogenannte Elektro-Akupunktur.

Bei modernen Akupunkturgeräten zur Durchführung dieser Elektroakupunktur wird üblicherweise der Körper mit der einen Elektrode eines Niederspannungs-Stromkreises verbunden. Zum schließen des Stromkreises dient eine punktförmige Elektrode, die auf den jeweiligen Akupunktur-Punkt aufgesetzt wird. Dadurch fließt der Strom über diesen Akupunktur-Punkt durch den Körper hindurch zur jenseitigen Elektrode, üblicherweise einem kleinen Metall-Hohlzylinder, der von der Patientenhand umschlossen wird. Zusätzlich kann in den Stromkreis noch ein Strommesser eingefügt sein, dessen Ausschlag erkennen läßt, ob tatsächlich auch durch die punktförmige Elektrode der Akupunktur-Punkt getroffen wurde. Der Nachteil dieses Verfahrens gegenüber demjenigen beim Einstechen von Nadeln ist darin zu erblicken, daS die Einwirkung nur kurzzeitig vor sich geht und daher zum Beeinflussen der Akupunktur-Punkte ein verhältnismäßig hoher Strom benötigt wird, der durchaus zur Irritation des dort befindlichen Gewebes fuhren kann. Es sind daher auch schon Punktelektroden bekannt, die diese Reizung durch elektrischen Strom ergänzen durch eine thermische Reizung. Bei dieser Sonderform der Punktelektrode wird die Elektrode auf eine verhältnismäßig hohe Temperatur gebracht, so daß gleichzeitig mit der Stromreizung eine länger andauernde Wärmereizung des jeweiligen Punktes stattfindet. Diese Behandlung ist allerdings schmerzhaft und unangenehm.

Eine Besonderheit der Akupunktur bildet die Ohr-Akupunktur, also die Stimulation von Triggerpunkten an der Ohrmuschel. Die Lehre dieser Ohr-Akupunktur besagt, daß sich nahezu sämtliche, ansonsten über die gesamte Körperoberfläche verteilten Akupunktur-Punkte am Ohr in diesen Triggerpunkten wiederfinden, so daß, statt die Akupunktur-Punkte am Körper zu reizen, der gleiche Effekt auch durch Reizung der zugeordneten Triggerpunkte an der Ohrmuschel stattfinden kann. Diese Lehre der Ohr-Akupunktur macht sich die Erfindung zunutze, die sich die Aufgabe gestellt hat eine Möglichkeit anzugeben, wie die altbewährte Akupunktur auf der Grundlage der Elektro-Akupunktur durchgeführt werden kann, ohne daß die oben beschriebenen Nachteile in Kauf genommen werden müssen. Insbesondere soll es möglich sein, die Einwirkungen des elektrischen Stromes über einen längeren Zeitraum aufrechtzuerhalten, um eine stete geringe Stimulierung der jeweiligen Punkte zu erreichen und damit ebenso stetig eine bessere Funktion des dem jeweiligen Akupunktur-Punkt zugeschriebenen Organs zu bewirken. Erreicht wird dies in erfindungsgemäßer Weise, ausgehend von einem kreolenförmigen, beim Tragen in die Ohrmuschelgrube einzuhängenden, das Ohrläppchen umgreifenden, an der Ohrmuschel-Rückseite anliegenden Ohrschmuck, der einseitig zum Einhängen in die Ohrmuschelgrube zapfenförmig ausgebildet ist dadurch, daß zumindest ein Anteil der beim Tragen des Ohrschmucks an der Haut anliegenden Ohrschmuckfläche als Kontaktfläche mit zumindest zwei elektrisch voneinander isolierten Kontakten ausgebildet ist und daß die Kontakte mit einer am (im) Ohrschmuck angebrachten Stromquelle verbunden sind.

Nicht mehr also wie bei der üblichen Ohr-Akupunktur werden Nadeln aus verschiedenen Materialien in die Ohrmuschel eingestochen und dort für einige Zeit belassen, oder es wird eine Reizung dieser Triggerpunkte durch kurzzeitiges Aufdrücken einer Elektrode im Wege der Elektroakupunktur erreicht, sondern es wird eine elektrische Reizung der jeweils gewünschten Triggerpunkte dadurch erreicht, daß ein Ohrschmuck getragen wird, der an den auf der Haut der Ohrmuschelgrube anliegenden Flächen Kontaktstellen aufweist, die mit einer im Ohrschmuck integrierten Stromquelle in Verbindung stehen. Dadurch fließt nicht nur über beliebig lange Zeit ein Strom über die betreffenden Kontakte zu den Triggerpunkten, sondern es ergibt sich auch bei entsprechender Ausbildung des Ohrschmucks, durch die stete Bewegung des Ohrschmucks beim Tragen, ein Überstreichen verschiedener Triggerpunkte, die auf diese Art und Weise alternierend stimuliert werden. Da zudem das Tragen von Ohrschmuck weltweit verbreitet ist, kommt noch hinzu, daß dieser "therapeutische" Ohrschmuck nicht nur den Träger beziehungsweise die Trägerin schmückt, sondern auch den therapeutischen Zweck nicht erkennen läßt, also berücksichtigt, daß derartige Gesundheitshilfen dem jeweiligen Betrachter nicht signalisiert werden. Hinsichtlich des fließenden Stromes ist darauf hinzuweisen, daß der Kontaktwiderstand, insbesondere in der Ohrmuschelgrübe, jedoch auch an den weiteren Hautstellen des Ohres, dadurch vermindert ist, daß sich dort infolge der Schweißproduktion stets Feuchtigkeit befindet, die den Kontaktwiderstand zwischen den Kontaktflächen des Ohrschmucks und der Haut sehr stark reduziert.

Die Stromquelle kann eine Primärzelle, vorzugsweise eine im Handel erhältliche Knopfzelle sein, die ihrer Kleinheit und des geringen Gewichtes wegen unschwer bei der erfindungsgemäßen Anordnung einzusetzen ist. Gleichfalls sehr vorteilhaft sind für diesen Verwendungszweck als flexible Folien ausgebildete Primärzellen zu verwenden. Die Stromquelle kann auch eine Solarzelle (Solarbatterie), vorzugsweise mit nachgeschalteter Sekundärzelle sein, da ja in aller Regel die Ohren frei getragen werden, also der Sonnen- beziehungsweise allgemein der Lichtbestrahlung zugänglich sind. Auch kann die Stromquelle eine Primär- oder Solarzelle sein, der ein Spannungsvervielfacher mit Schalter und/oder Impulsgeber nachgeschaltet ist, um, wenn dies notwendig ist, auch höhere Spannungen beziehungsweise Spannungsimpulse zu erzeugen. Da die Reizung der jeweiligen Triggerpunkte beziehungsweise Linien einhergeht mit der Größe der Spannung, kann durch die Wahl der Stromquelle Einfluß auf die jeweils gewünschte Stimulation genommen werden. Dies bezieht sich nicht nur auf die an die Kontakte angelegte Spannung, sondern auch auf die Stromart. So kann beispielsweise eine Stimulierung durch Gleichstrom erfolgen, wobei die Spannungswahl bereits durch die Batteriewahl zwischen 1 Volt und 4 Volt festge legt werden kann. Ein eingefügter Umschalter kann jeweils nach bestimmten Zeitabständen hierbei den Stromfluß umkehren. Weiter kann auch die Stimulierung mit Wechselspannung erfolgen, insbesondere dann wenn, wie dies bei manchen Akupunktur-Punkten angebracht ist, eine höhere Spannung gewünscht wird, die von der Batterie stammende Gleichspannung also sowieso zum Hochtransformieren zerhackt werden muß. Weiter besteht auch die Möglichkeit, die Kontakte mehr oder weniger weit voneinander anzuordnen, um dadurch ein mehr oder weniger tiefes Eindringen der Ströme in das jeweilige Gewebe und damit eine mehr oder weniger starke Reizung zu bewirken.

Auf der Zeichnung sind Ausführungsbeispiele des Neuerungsgegenstandes schematisch dargestellt, und zwar zeigen:
- Fig. 1: einen Ohrschmuck mit gegenüberliegenden Kontaktflächen,
- Fig. 2: einen Ohrschmuck mit einer punktförmigen Kontaktfläche und
- Fig. 3: eine Ansicht nach III der Fig. 2.

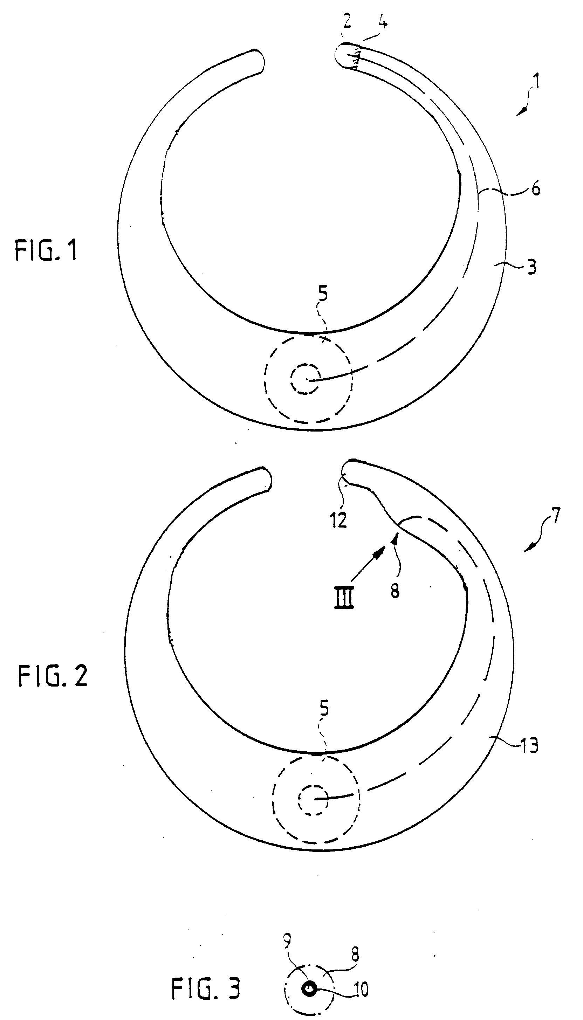

Bei einem kreolenförmigen Ohrschmuck (1) stehen sich die beiden Enden des Ohrschmucks so gegenüber, daß eines der Enden in die Ohrmuschelgrube eingesetzt werden kann und sich die Stirnseite des anderen Endes an der Rückseite der Ohrmsuchelgrube abstützt. Bei der Ausführung nach Fig. 1 ist das eine Ende (2) zapfenförmig ausgebildet und von dem restlichen Ohrschmuck-Körper (3) durch eine isolierende Zwischenlage (4) getrennt. Eingefügt in den Ohrschmuck (1) ist eine als Knopfzelle ausgeführte Batterie (5), die einerseits mit dem Ohrschmuckkörper (3) und andererseits über eine Drahtverbindung (6) mit dem Ende (2) des Ohrschmucks elektrisch verbunden ist. Beim Tragen dieses Ohrschmucks (1) fließt, ausgehend von dem einen Pol der Batterie (5), der mit dem Ende (2) verbunden ist, ein Strom von diesem Ende (2) durch das dazwischen befindliche Ohrmuschel-Gewebe hindurch zum anderen Ende des Ohrschmucks (1) oder auch, falls der Ohrschmuckkörper (3) an anderen Hautteilen anliegt, von diesem Ende (2) jeweils zu den Berührungsstellen des Ohrschmuckkörpers (3) mit dem Ohrmuschelgewebe. Hierdurch werden insbesondere die in der Ohrmuschelgrube befindlichen Triggerpunkte stimuliert beziehungsweise werden dadurch die gewünschten Wirkungen erzielt.

Bei der Ausführung eines Ohrschmucks (7) nach Fig. 2 befindet sich eine Kontaktfläche (8) an der Innenseite des Ohrschmuckkörpers (13), unterhalb des Einhängepunktes in die Ohrmuschelgrube über das Ende (12) des Ohrschmuckkörpers (13). Dadurch werden dort befindliche Triggerpunkte stimuliert, da nun ein Strom von dem mit der Batterie (5) verbundenen Kontakt (9), der durch eine Isolierung (10) getrennt in den Ohrschmuckkörper (13) eingefügt ist, fließt. Wie dieses Beispiel zeigt, können beliebige Triggerpunkte durch jeweilige Kontaktflächenhöcker (8) an der Innenseite der Kreole erreicht werden.

## Patentansprüche

1. Kreolenförmiger, beim Tragen in die Ohrmuschelgrube einzuhängender, das Ohrläppchen umgreifender, an der Ohrmuschel-Rückseite anliegender Ohrschmuck, der einseitig zum Einhängen in die Ohrmuschelgrube zapfenförmig ausgebildet ist,
dadurch gekennzeichnet,
daß zumindest ein Anteil der beim Tragen des Ohrschmucks (1, 7) an der Haut anliegenden Ohrschmuckfläche als Kontaktfläche (8) mit zumindest zwei elektrisch voneinander isolierten Kontakten (9) ausgebildet ist und daß die Kontakte (9) mit einer am (im) Ohrschmuck (1, 7) angebrachten Stromquelle (5) verbunden sind.

2. Ohrschmuck nach Anspruch 1,
dadurch gekennzeichnet,
daß die Stromquelle (5) eine Primärzelle ist.

3. Ohrschmuck nach Anspruch 1,
dadurch gekennzeichnet,
daß die Stromquelle eine Solarzelle (Solarbatterie), vorzugsweise mit nachgeschalteter Sekundärzelle ist.

4. Ohrschmuck nach Anspruch 1,
dadurch gekennzeichnet,
daß die Stromquelle (5) eine Primär- oder Solarzelle mit nachgeschaltetem spannungsvervielfacher mit Schalter und/oder Impulsgeber ist.

5. Ohrschmuck nach Anspruch 1,
dadurch gekennzeichnet,
daß der Ohrschmuck (1, 7) aus elektrisch leitendem Material, vorzugsweise aus Metall ist, daß die Stromquelle (5) einpolig mit diesem als Kontakt dienenden Material verbunden ist und daß der zweite Kontakt (9) isoliert (4, 10) in dieses Material eingefügt ist.

6. Ohrschmuck nach Anspruch 1 oder 5,
dadurch gekennzeichnet,
daß der Ohrschmuck (1, 7) mehrere, beim Tragen an der Haut anliegende, kontaktbestückte Ohrschmuckflächen aufweist.

7. Ohrschmuck nach Anspruch 1 oder 5,
dadurch gekennzeichnet,
daß das elektrisch leitende, zapfenförmige Ende (2) des Ohrschmucks (1) vom restlichen elektrisch leitenden, einpolig mit der Stromquelle (5) verbundenen Ohrschmuckkörper (3) isoliert (4) und einpolig mit der Stromquelle (5) verbunden ist.
